# EUROPÄISCHE PATENTSCHRIFT

(11) **EP 0 913 385 B1**
(45) Veröffentlichungstag und Bekanntmachung des Hinweises auf die Patenterteilung: **07.08.2002**
(21) Anmeldenummer: 98119467.3
(22) Anmeldetag: 15.10.1998
(51) Int. Cl.: C07C 59/265, C07C 51/42, C07C 51/47, C07C 51/48

(54) **Verfahren zur Herstellung von Citronensäure und/oder Citraten**
Process for the preparation of citric acid and/or citrates
Procédé de préparation d'acide citrique et/ou de citrates

(30) Priorität: 30.10.1997 DE 19747902
(43) Veröffentlichungstag der Anmeldung: 06.05.1999
(73) Patentinhaber: MG Technologies AG, 60325 Frankfurt am Main (DE)
(72) Erfinder: Boensch, Rudolf Dr., 55299 Nackenheim (DE); Hohmann, Klaus, 65719 Hofheim (DE); Kuhn, Juergen, 65929 Frankfurt am Main (DE); Cerny, Vaclav, 33151 Kaznejov (CZ); Hotek, Frantisek, 43985 Petrohrad (CZ); Pendl, Jiri, 32322 Pilzen (CZ)
(74) Vertreter: Revesz, Veronika

(56) Entgegenhaltungen:
- EP-A- 0 151 470
- DD-A- 203 533
- DE-C- 19 545 303
- US-A- 5 237 098

## Beschreibung

Die Erfindung betrifft ein Verfahren zur Herstellung von Citronensäure und/oder deren Salze aus einem kohlehydrathaltigen Rohstoff, wobei man den Rohstoff reinigt, eine wässrige Lösung des Rohstoffs durch Fermentation in eine erste citronensäurehaltige Lösung umwandelt, die erste Lösung durch eine Zellabtrennung führt und eine zweite citronensäurehaltige Lösung gewinnt, die zweite Lösung bei Temperaturen von 2 bis 70 °C einer Proteinfällung unterwirft, in einer Filtration Protein abtrennt und eine dritte citronensäurehaltige Lösung erhält, welche man durch Ionenaustausch reinigt, anschließend entfärbt und aus der entfärbten Lösung Wasser mindestens teilweise entfernt.

Ein solches Verfahren ist aus DE 195 45 303 C1 bekannt. Dieses Patent beschreibt die Herstellung einer organischen Säure und/oder ihrer Salze und insbesondere von Milchsäure, Citronensäure oder Gluconsäure. Man führt hierbei das Sediment der Proteinfällung zurück in die Zellabtrennung, auch gewinnt man beim Konzentrieren des Produkts durch Kristallisation eine Mutterlauge, die man in die Proteinfällung zurückleitet.

Aus DD-A-203 533 ist ein ähnliches Verfahren zum Erzeugen aliphatischer Carbonsäuren bekannt, wobei man die Rohlösung nach der mechanischen Reinigung zunächst über einen stark sauren Kationenaustauscher führt und einen Teilstrom der Lösung einem Anionenaustauscher in der Carbonsäureform aufgibt. Beim Verfahren des US-Patents 5 237 098 wird Citronensäure aus Melasse gewonnen, wobei nach der Fermentation zunächst ein ein sekundäres oder tertiäres Amin enthaltendes organisches Lösungsmittel zugegeben wird, bevor man mittels Anionenaustauscher in Basenform Citronensäure adsorbiert.

Der Erfindung liegt die Aufgabe zu Grunde, das eingangs genannte Verfahren zu vereinfachen, es auf die Herstellung von Citronensäure und/oder Citrat auszurichten und dabei ein weitgehend reines Produkt möglichst kostengünstig zu erzeugen. Erfindungsgemäß gelingt dies durch die Merkmalskombination des Patentanspruchs 1.

Es ist zweckmäßig, dass man durch Eluieren des zweiten Anionenaustauschers mit Hydroxid eine vierte, Citrat enthaltende Lösung erhält, welche man entfärbt und aus welcher man z.B. in einem Kationenaustauscher Kationen entfernt und Citronensäure erhält, bevor man aus der vierten Lösung Wasser mindestens teilweise abtrennt. Wenn man demgegenüber beim Eluieren des zweiten Anionenaustauschers mit Wasser und vorzugsweise heißem Wasser arbeitet, erhält man eine Citronensäure-Lösung.

Es ist wichtig, den ersten Anionenaustauscher im Anfangszustand mit Citronensäure zu beladen, um die Reinheit der aus diesem Anionenaustauscher abfließenden Lösung weiter zu steigern. Ferner kann man die aus dem zweiten Anionenaustauscher kommende Lösung mit einem pH-Wert von 5 bis 9 und vorzugsweise 6 bis 8 in und durch ein Festbett aus körniger Aktivkohle leiten, in welchem die Lösung entfärbt wird. Die Aktivkohle dient hierbei insbesondere der Zuckerentfernung.

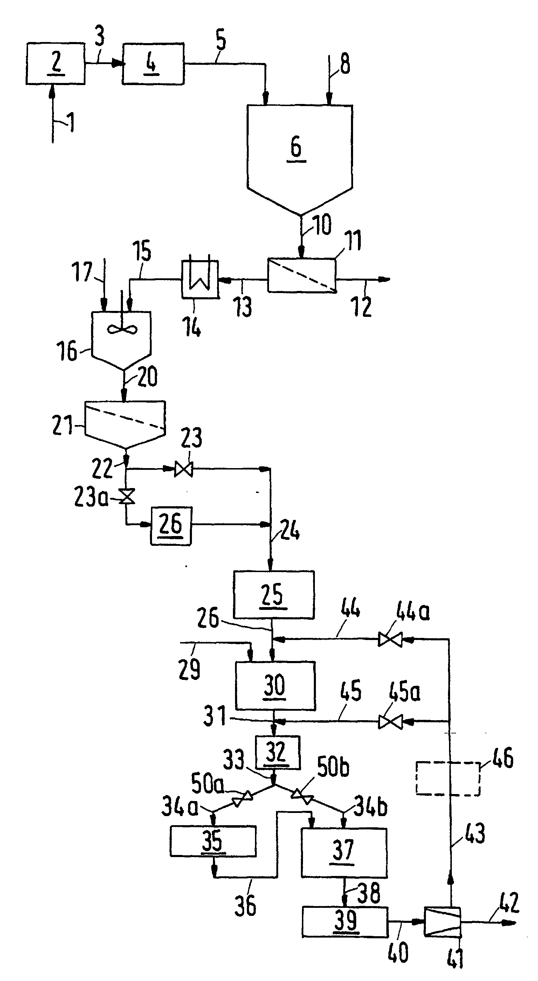

Weitere Ausgestaltungsmöglichkeiten des Verfahrens werden mit Hilfe der Zeichnung erläutert. Die Zeichnung zeigt ein Fließschema des Verfahrens.

Als Ausgangsmaterial dient eine wäßrige Glucose-, Saccharose- oder Stärkelösung. Man geht hierbei zum Beispiel von enzymatisch verflüssigter Stärke, verdünntem Glucosesirup oder aufgelöster kristalliner Glucose oder Saccharose aus. Die Lösung wird in der Leitung (1) herangeführt und zunächst einer Reinigung (2) unterzogen. Die Reinigung kann eine Aktivkohlesäule zum Entfärben und zusätzlich einen Kationen- und/oder Anionenaustauscher enthalten. Die gereinigte Lösung wird durch die Leitung (3) einer kontinuierlichen Sterilisation (4) oder Pasteurisierung unterzogen. Die Zucker- oder Stärkelösung wird dann in der Leitung (5) einer Fermentation (6) aufgegeben, wobei es zusätzlich zweckmäßig sein kann, an sich bekannte Fermentationshilfsmittel zuzudosieren. Die Flüssigkeit in der Fermentation (6) wird mit Pilzsporen (Aspergillus niger) geimpft, die in der Leitung (8) herangeführt werden. Die Fermentation kann ein- oder mehrstufig durchgeführt werden.

Durch die Fermentation gewinnt man eine erste Lösung, die man in der Leitung (10) zu einer Zellabtrennung (11) führt. Hier werden die Mycele z. B. mit Hilfe eines Vakuumbandfilters oder aber durch Zentrifugieren abgetrennt und in der Leitung (12) entfernt. Die weitgehend zellfreie Lösung, die hier als "zweite Lösung" bezeichnet wird, zieht man in der Leitung (13) ab und führt sie zur Desaktivierung pilzeigener Proteasen durch eine Kurzzeiterhitzung (14). Die Lösung wird hier üblicherweise 1 bis 5 Minuten lang auf etwa 75°C gehalten. Die so behandelte zweite Lösung wird durch die Leitung (15) einer Proteinfällung (16) aufgegeben. Die Lösung der Leitung (15) enthält üblicherweise 10 bis 25 Gew.-% Citronensäure und neben anderen gelösten organischen und anorganischen Bestandteilen noch eine geringe Menge an Zellbruchstücken.

In der Proteinfällung (16) kann es zweckmäßig sein, die Lösung im Chargenbetrieb mit einer wäßrigen SiO₂-Lösung zu versetzen, die aus der Leitung (17) kommt. Man verwendet z. B. SiO₂ mit einer BET-Oberfläche von 300 bis 500 m²/g, wobei die Lösung der Leitung (17) 20 bis 40 Gew.-% SiO₂ enthält. Pro m³ in der Leitung (15) herangeführter Lösung werden üblicherweise 0,1 bis 10 l SiO₂-Lösung als Fällungshilfsmittel durch die Leitung (17) zudosiert. Bei der Proteinfällung arbeitet man vorzugsweise bei Temperaturen im Bereich von 40 bis 60°C. Nach Reaktionszeiten von 0,5 bis 4 Stunden führt man die erzeugte Suspension in der Leitung (20) zu einer Filtrationseinrichtung (21).

Die Filtrationseinrichtung (21) arbeitet vorzugsweise mehrstufig und weist auch eine Ultrafiltration zum Entfernen restlicher Proteine auf. Eine gereinigte Lösung, die hier auch als "dritte Lösung" bezeichnet wird, zieht man in der Leitung (22) ab und kann sie direkt über das geöffnete Ventil (23) in der Leitung (24) zu einem ersten Anionenaustauscher (25) führen. Es kann aber statt dessen empfehlenswert sein, die dritte Lösung zunächst durch das geöffnete Ventil (23a) durch eine Entfärbung (26) zu führen, bevor man sie dem ersten Anionenaustauscher (25) aufgibt. Für die Entfärbung (26) verwendet man üblicherweise ein Aktivkohle-Festbett.

Der erste Anionenaustauscher (25) dient vor allem der Abtrennung anorganischer Anionen aus der dritten citronensäurehaltigen Lösung. Hierbei ist es wichtig, ein Anionenaustauschmaterial zu verwenden, das im Anfangszustand mit Citronensäure beladen ist. Während des Durchlaufens der dritten Lösung wird diese Citronensäure zumindest teilweise an die Lösung abgegeben und so deren Reinheit weiter gesteigert. Die aus dem ersten Anionenaustauscher in der Leitung (26) ablaufende Lösung führt man durch einen zweiten Anionenaustauscher (30). Im zweiten Anionenaustauscher (30) wird die gelöste Citronensäure selektiv gebunden, während störende Kationen und Restzucker sowie Aminosäuren nicht gebunden werden und den zweiten Anionenaustauscher in einer nicht dargestellten Leitung verlassen. Der zweite Anionenaustauscher (30) kann z. B. in der OH-Form vorliegen oder freie Ammoniumgruppen enthalten, damit er Citronensäure binden kann.

Wenn der zweite Anionenaustauscher (30) mit Citronensäure voll beladen ist, wird diese in einem separaten Verfahrensschritt eluiert. Zum Eluieren kann man eine Hydroxid-Lösung oder auch Wasser verwenden, wobei die Elutionsflüssigkeit durch die Leitung (29) herangeführt wird. Enthält die Elutionslösung NaOH, so ist in der ablaufenden Lösung der Leitung (31) Na-Citrat gelöst. Die Lösung der Leitung (31) wird hier auch als "vierte Lösung" bezeichnet. Beim Eluieren mit Wasser ist die vierte Lösung frei von Natrium und enthält Citronensäure.

Die vierte Lösung der Leitung (31) wird zunächst durch eine Entfärbung (32) (z. B. Aktivkohle) geführt und fällt in der Leitung (33) als entfärbte Lösung an. Wenn die Lösung Anionen, z. B. Na⁺, enthält, die im Produkt stören würden, führt man die Lösung durch das geöffnete Ventil (50a) und die Leitung (34a) zu einem Kationenaustauscher (35), der diese Anionen bindet. An die Stelle des Kationenaustauschers (35) kann auch eine Elektrodialyse treten. Anschließend wird die Lösung durch die Leitung (36) einer Konzentrierung und Eindampfung (37) aufgegeben. Wenn die vierte Lösung der Leitung (33) jedoch keine störenden Anionen enthält oder aber Citrat als Produkt gewonnen werden soll, führt man die Lösung durch das geöffnete Ventil (50b) und die Leitung (34b) direkt in die Konzentrierung und Eindampfung (37). Konzentrierte Flüssigkeit zieht man in der Leitung (38) ab, und gibt sie einer Kristallisation (39) auf. Die gewonnene kristallhaltige Suspension gelangt in der Leitung (40) zu einer Zentrifuge (41), aus welcher man das Produkt, Citronensäure oder Citrat in reiner, kristalliner Form, in der Leitung (42) abzieht. Die Mutterlauge wird in der Leitung (43) abgeführt und entweder durch das geöffnete Ventil (44a) und die Leitung (44) dem zweiten Anionenaustauscher (30) oder durch das geöffnete Ventil (45a) und die Leitung (45) der Entfärbung (32) aufgegeben. Falls nötig, wird die Flüssigkeit der Leitung (43) noch durch einen Kationenaustauscher (46) geleitet, um eine höhere Reinheit zu erreichen.

### Beispiel:

In einer der Zeichnung entsprechenden Verfahrensführung, in welcher die Ventile (23a), (50a) und (44a) geschlossen sind, wird Na-Citrat von Pharmaqualität hergestellt. Hierbei wird gereinigte und sterilisierte Zuckerlösung durch die Leitung (5) in die Fermentation (6) gegeben. Die Lösung besteht aus 1,2 t Zucker, die in 5,9 t Wasser gelöst sind. Es wird mit einem Blasensäulenfermenter gearbeitet, die Temperatur beträgt 32°C. Dem Fermenter werden 100 g Pilzsporen Aspergillus niger zudosiert; die Fermentation wird unter Belüftung 5 Tage lang durchgeführt. Die anschließende Zellabtrennung (11) erfolgt mit Hilfe eines Vakuum-Bandfilters, bei der Kurzzeit-Erhitzung (14) wird die 2. Lösung 7 Minuten lang auf 80°C gehalten. Pro Liter enthält die Lösung der Leitung (15) 0,5 g Zucker, 200 g Citronensäure, 5 g gelöste Proteine, 2,4 g Kationen und 2,5 g Anionen. Pro m³ Lösung der Leitung (15) gibt man in der Proteinfällung (16) 0,5 1 SiO₂-Lösung zu, sie enthält 70 Gew.-% SiO₂, die in Wasser kolloidal gelöst sind.

Nach kurzem Verrühren der Lösungen aus den Leitungen (15) und (17) hat die sich in der Fällung (16) bildende Suspension 5 Stunden Zeit zum Absetzen des Proteinschlamms, der dann als Filtrationshilfsmittel zur Zellabtrennung (11) zurückgeführt wird. Die weitgehend klare, citronensäurehaltige Flüssigkeit wird durch die Leitung (20) zur Ultrafiltration (21) geleitet, wo restliches Protein entfernt wird. Durch das geöffnete Ventil (23) gelangt die filtrierte Flüssigkeit zum ersten Anionenaustauscher (25), der im Anfangszustand mit Citronensäure beladen ist, und dann zum zweiten Anionenaustauscher (30), der sich in der OH-Form befindet. Für beide Austauscher werden 5 m³ Lewatit (Bayer) verwendet. Bei der Elutionsflüssigkeit der Leitung (29) handelt es sich um eine wäßrige NaOH-Lösung mit 10 Gew.-% NaOH. Die in der Leitung (31) abgezogene 4. Lösung enthält 170 g/l Tri-Na-Citrat sowie Spuren von Zucker und organischen Verunreinigungen, der pH-Wert beträgt 7,5. Die Entfärbung (32) arbeitet mit einem Festbett von Aktivkohle (EPIBON von Lurgi) mit einem Volumen von 5 m³. Durch die Leitung (34b) wird die Lösung der Konzentrierung und Eindampfung (37) zugeführt, die mit Umlaufverdampfern arbeitet. Die Lösung in der Leitung (38), die zum kontinuierlich arbeitenden Kristallisator (39) führt, enthält 960 g/l Tri-Na-Citrat, und die Suspension in der Leitung (40) enthält 1200 g/l Tri-Na-Citrat. Die Mutterlauge wird durch die Leitung (43) ohne den Kationenaustauscher (46) durch das geöffnete Ventil (45a) und durch die Leitung (45) zum Entfärber (32) zurückgeleitet.

## Patentansprüche

1. Verfahren zur Herstellung von Citronensäure und/oder deren Salze aus einem kohlehydrathaltigen Rohstoff, wobei man den Rohstoff reinigt, eine wässrige Lösung des Rohstoffs durch Fermentation in eine erste citronensäurehaltige Lösung umwandelt, die erste Lösung durch eine Zellabtrennung führt und eine zweite citronensäurehaltige Lösung gewinnt, die zweite Lösung einer Proteinfällung bei Temperaturen von 2 bis 70°C unterwirft, in einer Filtration (21) Protein abtrennt, **dadurch gekennzeichnet, dass** aus der Filtration eine dritte citronensäurehaltige Lösung abgezogen wird, dass die dritte Lösung durch einen ersten Anionenaustauscher (25) geleitet wird, der im Anfangszustand mit Citronensäure beladen ist, dass die aus dem ersten Anionenaustauscher abfließende citronensäurehaltige Lösung durch einen zweiten Anionenaustauscher (30) geleitet wird, welcher mit Citronensäure selektiv beladen ist, dass der mit Citronensäure beladene zweite Anionenaustauscher mit Hydroxid-Lösung oder Wasser eluiert wird und die vierte Lösung erhält, in der Citrat oder Citronensäure gelöst ist, und dass aus der vierten Lösung nach einer Entfärbung Wasser mindestens teilweise entfernt wird.

2. Verfahren nach Anspruch 1, **dadurch gekennzeichnet, dass** durch Eluieren des zweiten Anionenaustauschers mit Hydroxid eine vierte, Citrat enthaltende Lösung hergestellt wird, welche entfärbt wird und aus der die Kationen entfernt werden und Citronensäure erhält, bevor aus der vierten Lösung Wasser mindestens teilweise abtrennt wird.

3. Verfahren nach Anspruch 1 oder 2, **dadurch gekennzeichnet, dass** die aus der Filtration kommende dritte Lösung einer Entfärbungsbehandlung unterzogen wird, bevor die dritte Lösung durch den ersten Anionenaustauscher geleitet wird.

4. Verfahren nach Anspruch 1 oder einem der folgenden, **dadurch gekennzeichnet, dass** die aus dem zweiten Anionenaustauscher kommende Lösung mit einem pH-Wert von 5 bis 9 in ein Festbett aus körniger Aktivkohle geleitet wird, in welcher die Lösung entfärbt wird.

## Claims

1. A process of producing citric acid and/or the salts thereof from a raw material containing carbohydrates, wherein the raw material is purified, an aqueous solution of the raw material is converted into a first solution containing citric acid by means of fermentation, the first solution is passed through a cell separation stage and a second solution containing citric acid is obtained, the second solution is subjected to protein precipitation at temperatures of 2 to 70°C, protein is separated off in a filtration stage (21), **characterised in that** a third solution containing citric acid is withdrawn from the filtration stage, the third solution is passed through a first anion exchanger (25) which in the initial state is laden with citric acid, that the solution containing citric acid which is discharged from the first anion exchanger is passed through a second anion exchanger (30) which is selectively charged with citric acid, that the second anion exchanger charged with citric acid is eluted with hydroxide solution or water and a fourth solution is obtained in which citrate or citric acid is dissolved, and that water is at least partly removed from the fourth solution after decolourisation.

2. The process according to claim 1, **characterised in that** a fourth solution containing citrate is obtained, which solution is decolourised by eluting the second anion exchanger with hydroxide, and from which the cations are removed and citric acid is obtained, before water is at least partly separated from the fourth solution.

3. The process according to claim 1 or 2, **characterised in that** the third solution coming from the filtration stage is subjected to a decolourisation treatment before the third solution is passed through the first anion exchanger.

4. The process according to claim 1 or one of the preceding claims, **characterised in that** the solution coming from the second anion exchanger with a pH value of 5 to 9 is introduced into a fixed bed of granular activated carbon in which the solution is decolourised.

## Revendications

1. Procédé de préparation d'acide citrique et/ou de ses sels à partir d'une matière brute contenant de l'hydrate de carbone, qui consiste à purifier la matière brute, à transformer une solution aqueuse de la matière brute par fermentation en une première solution contenant de l'acide citrique, à envoyer la première solution dans une séparation de cellules et à obtenir une deuxième solution contenant de l'acide citrique, à soumettre la deuxième solution à une précipitation de protéines à des températures de 2 à 70°C, à séparer de la protéine dans une filtration (21), **caractérisé en ce qu'**il consiste à soutirer de la filtration une troisième solution contenant de l'acide citrique, à envoyer la troisième solution dans un premier échangeur (25) d'anions, qui est chargé à l'état initial d'acide citrique, à envoyer la solution contenant de l'acide citrique sortant du premier échangeur d'anions dans un deuxième échangeur (30) d'anions, qui est chargé sélectivement d'acide citrique, à éluer par une solution d'hydroxyde ou par de l'eau le deuxième échangeur d'anions chargé d'acide citrique et à obtenir la quatrième solution, dans laquelle du citrate ou de l'acide citrique est dissous et à éliminer au moins en partie de la quatrième solution de l'eau après une décoloration.

2. Procédé suivant la revendication 1, **caractérisé en ce qu'**il consiste à préparer par élution du deuxième échangeur d'anions par de l'hydroxyde une quatrième solution contenant du citrate, que l'on décolore et dont on élimine les cations et dont on obtient de l'acide citrique avant de séparer au moins en partie de l'eau de la quatrième solution.

3. Procédé suivant la revendication 1 ou 2, **caractérisé en ce qu'**il consiste à soumettre la troisième solution provenant de la filtration à un traitement de décoloration avant d'envoyer la troisième solution dans le premier échangeur d'anions.

4. Procédé suivant la revendication 1 ou l'une des suivantes, **caractérisé en ce qu'**il consiste à envoyer la solution provenant du deuxième échangeur d'anions et ayant un pH de 5 à 9 dans un lit fixe de charbon actif en grain dans lequel la solution est décolorée.
